(19) Europäisches Patentamt
European Patent Office
Office européen des brevets

(11) **EP 3 788 887 A1**

(12) **EUROPEAN PATENT APPLICATION**
published in accordance with Art. 153(4) EPC

(43) Date of publication:
**10.03.2021 Bulletin 2021/10**

(21) Application number: **19796803.5**

(22) Date of filing: **03.05.2019**

(51) Int Cl.:
*A23L 33/105* [(2016.01)]   *A23L 19/00* [(2016.01)]
*A23F 5/24* [(2006.01)]

(86) International application number:
**PCT/KR2019/095002**

(87) International publication number:
**WO 2019/212327 (07.11.2019 Gazette 2019/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.05.2018 KR 20180051743**
**02.05.2019 KR 20190051477**

(71) Applicant: **Noahs Co., Ltd.**
**Gyeonggi-do 12101 (KR)**

(72) Inventors:
• **PARK, Ji-Ho**
**Seoul 04004 (KR)**
• **LEE, Yoon Jung**
**Seoul 03924 (KR)**

(74) Representative: **Cabinet Chaillot**
**16/20, avenue de l'Agent Sarre**
**B.P. 74**
**92703 Colombes Cedex (FR)**

<u>Remarks:</u>
The applicant has filed a text with which it is intended to bring the translation into conformity with the application as filed (Art. 14(2) EPC).

(54) **COMPOSITION FOR IMPROVING SLEEP DISORDERS CAUSED BY CAFFEINE CONTAINING CITRUS RIND EXTRACT**

(57)   The present invention relates to a composition for improving sleep disorders caused by caffeine containing a citrus rind extract as an active ingredient.

[FIG. 12]

EP 3 788 887 A1

**Description**

**[Technical Field]**

The present invention relates to a composition for improving sleep disorders caused by caffeine containing a citrus rind extract as an active ingredient.

**[Background Art]**

Caffeine is a kind of alkaloid contained in the fruits, leaves, and seeds of some plants, and is contained in coffee bean seeds, tea leaves, cacao, cola fruits, and the like.

The caffeine is contained in various foods such as coffee, chocolate, cola, tea, soft drinks, tonic drinks and medicines, and the coffee is being spotlighted as the most popular beverage among foods containing caffeine.

The coffee contains approximately 50 to 150 mg of caffeine per 200 mL. Since the caffeine contained in coffee exhibits effects such as fatigue recovery and arousal, modern people tired of stress and fatigue drink more than one cup of coffee a day.

However, when a person takes a large amount of caffeine or a person with a weak mind and body takes caffeine, anxiety, nervousness, insomnia, headache, heart tremor, palpitations, etc. may occur (Korean Patent Registration No. 10-1265253).

Therefore, many studies have been conducted to develop new food and beverage products capable of minimizing side effects such as sleep disorders caused by caffeine, but there is still no way to supplement the side effects caused by caffeine while maintaining the caffeine content to satisfy the public's preference.

**[Disclosure]**

**[Technical Problem]**

An object of the present invention is to provide a food composition for improving sleep disorders caused by caffeine containing a citrus rind extract.

Another object of the present invention is to provide a pharmaceutical composition for preventing and treating sleep disorders caused by caffeine containing a citrus rind extract.

**[Technical Solution]**

In order to achieve the objects, an aspect of the present invention relates to a food composition for improving sleep disorders caused by caffeine containing a citrus rind extract.

The dictionary meaning of *"Citrus"* is a generic name over the three genera of *Citrus, Fortunella,* and *Pontirus* of the Citrus family, and includes various compounds such as narigin, naringenin, hesperidin, tangeretin, and nobiletin.

The citrus refers to fruits and immature fruits thereof included in the citrus series such as clementine, kumquat, mandarin, tangerine, sweetie, citron, citrus sudachi, cheonhyehyang, calamansi, pomelo, hanrabong, orange, lemon, grapefruit, lime, yuja, etc., and is not particularly limited as long as the citrus belongs to the Citrus plant.

The naringin contained in the citrus is a glycoside of flavonoid pigments and has excellent antibacterial activity due to strong bacteriostatic and antibacterial effects through antioxidant, metal-blocking, and ascorbic acid synergic effects.

The naringenin is known to have anti-inflammatory, antioxidant, and anticancer activities as well as antibacterial and antiviral activities.

The hesperidin is known to lower blood pressure and blood sugar through lipid degradation, and to lower a blood LDL cholesterol level through reinforcement of capillaries and improvement of fat metabolism.

The tangeretin is known to have anti-inflammatory, antioxidant, and anticancer activities with a neuroprotective action.

The nobiletin is effective as a therapeutic agent for asthma by regulating the lipid metabolism and relaxing the bronchial smooth muscle, and is known to have an effect of preventing heart disease.

The "rind" refers to the peel covering the outer side of the pulp, and is divided into an exocarp, a mesocarp, and an endocarp in the botanical meaning, and the citrus rind according to the present invention refers to an exocarp and a mesocarp. Specifically, the citrus rind of the present invention may be a citrus unshiu peel, but is not limited thereto.

In the present invention, the *"citrus unshiu* peel" is a medicinal herb defined as the peel of a ripe fruit of a clementine tree, and refers to a dried rind. In the present invention, the citrus unshiu peel and the rind may be used in combination.

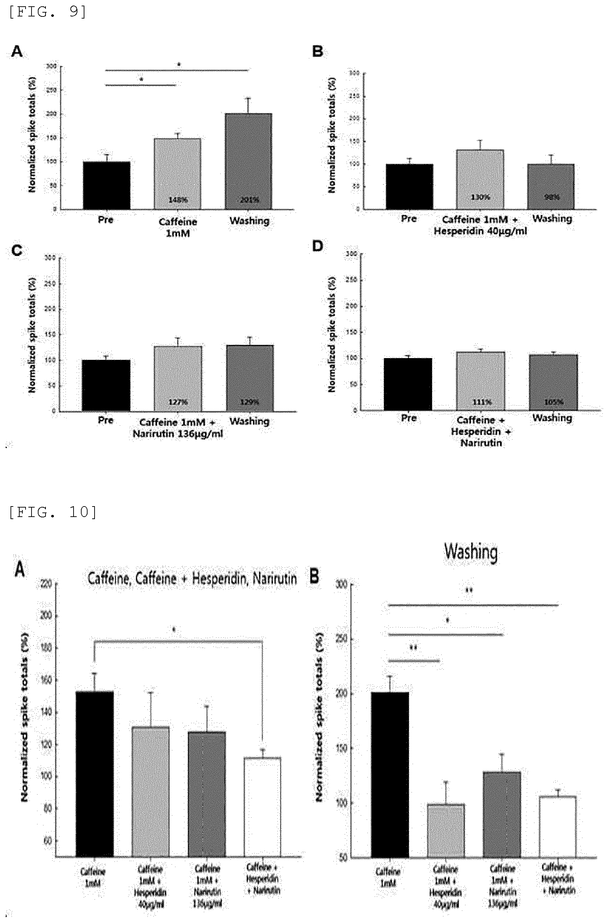

In one embodiment of the present invention, it was confirmed that the neural activity caused by caffeine was alleviated or inhibited when hesperidin, narirutin, or a mixture thereof was treated (FIGS. 9 and 10). Specifically, the citrus rind extract may contain at least one of hesperidin and narirutin.

In the present invention, the "extract" refers to a substance obtained by isolating an active ingredient contained in an extract material by contacting a solvent and the extract material under specific conditions, and the extract may contain an active ingredient in a raw material by an extraction process for the citrus rind extract.

The citrus rind extract may be extracted using a general solvent according to a general method known in the art for extracting an extract from a natural product, that is, under conditions of general temperature and pressure.

Specifically, the citrus rind extract may be extracted by using water, alcohol, ethyl acetate, acetone, nucleic acid, dichloromethane, or a mixed solvent thereof, and more specifically, water may be used.

The extraction method may use various methods, such as hot water extraction, cold needle extraction, reflux extraction, and ultrasonic extraction, and preferably hot water extraction, but is not limited thereto.

The caffeine (1,3,7-trimetnylxanthine) of the present invention is present in coffee trees, cacao fruits, etc. as a psychotropic substance widely used as a central nervous system stimulant, and is also contained in cola, chocolate, etc. The caffeine is a substance that acts as an inhibitor of binding of adenosine and adenosine receptors (adenosine A1 receptor and adenosine A2a receptor).

The adenosine is a neuromodulator and a substance that promotes sleep and suppresses arousal by inhibiting the production of neurotransmitters and promoting the production of gamma-aminobutyric acid (GABA).

The GABA (gamma-aminobutyric acid) is associated with effects of anxiety relief, prevention or improvement of convulsion, sedation, and induction and improvement of sleep, and sleep disorders may occur by inactivation of GABA.

When the GABA and the caffeine act together, the effect of caffeine is better, and the adenosine receptor is inhibited by caffeine, and the inhibited adenosine receptor overexpresses the neurotransmitters and inhibits the production of GABA.

Accordingly, proper intake of caffeine stimulates the central and peripheral nervous systems to help in improving concentration and preventing drowsiness, but excessive intake of caffeine causes sleep disorders such as nervous irritability, excitement or insomnia, and may also affect the endocrine system.

The citrus rind extract acts competitively with caffeine to antagonize the adenosine receptor, thereby inhibiting the production of neuroactive substances and effectively increasing the production of GABA so as to implement an anti-insomnia effect. The "antagonism" means enclosing and competing for a limited specific binding site between similar substances.

In one embodiment of the present invention, as a result of confirming whether a different type of citrus rind extract other than the citrus unshiu peel has an effect of alleviating neural activity, it was confirmed that the degree of neural activity was reduced to a level similar or equivalent to that of the citrus unshiu peel extract.

In addition, specifically, the citrus rind extract may prevent or improve sleep disorders caused by caffeine, and more specifically, may minimize sleep disorders caused by caffeine.

In the present invention, the "sleep" is an important physiological function of animals with developed brain, and refers to a behavior indispensable for survival. The brain may exert the ability capable of processing high-level information due to good sleep.

In the present invention, the "sleep disorder" means a condition characterized by waking up without recovery, waking up in the middle of the night, having difficulty in falling asleep again after waking up, having difficulty in falling asleep, waking up too early, etc., and is a disease reported to affect about 15% of all adults per year in Korea.

The sleep disorder has been widely used as meaning any and all types and grades of sleep loss. The sleep disorder may last either acute or chronic.

The sleep disorder may include all types of sleep disorders known in the art, and may be sleep disorders caused by stress, health conditions, pain, medication, jet lag, noise, or the like, and specifically, sleep disorders caused by caffeine.

In the present invention, the "prevention" means all actions that inhibit or delay the occurrence of sleep disorders in advance. The prevention may be complete or partial. In this case, the prevention may mean a phenomenon in which the degree of sleep disorder of a subject decreases compared to a case where the composition for preventing or improving the sleep disorders is not used.

The "improvement" of the present invention means all actions that at least reduce parameters associated with conditions to be treated, e.g. the degree of symptoms. The improvement includes all actions in which the symptoms of sleep disorders are improved or beneficially changed by using the citrus rind or citrus unshiu peel extract of the present invention.

Since the citrus rind extract uses a substance derived from a natural product as it is, unlike chemical substances, fatal side effects caused by intake or administration thereof may be minimized.

More specifically, the sleep disorder may be insomnia or circadian rhythm sleep disorders caused by caffeine.

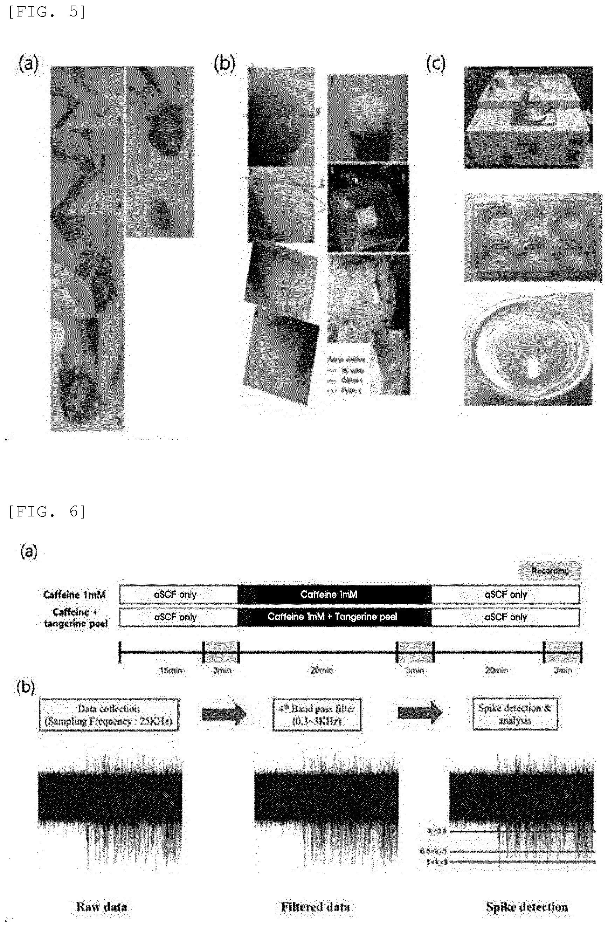

In the present invention, the "insomnia" refers to a condition where symptoms of having trouble sleeping, awakening frequently while sleeping, or showing unpleasant when waking up in the morning are shown alone or in combination. It is known that the insomnia may occur when taking caffeine, and it has been confirmed that the composition of the present invention improved the reduction of sleep duration caused by caffeine (FIG. 6), and thus, the composition of the present invention may be used for improving the insomnia.

In the present invention, the "circadian rhythm sleep disorders" are disorders that occur when a problem occurs in the body organs taking charge of the circadian rhythm or the alignment between the internal circadian rhythm and external factors is broken. Representative diseases include delayed or advanced sleep phase syndromes, etc., and may occur when a biological cycle is changed due to the influence of physiological or neurological activity by compounds such as caffeine. It was confirmed that the composition of the present invention may alleviate the neural activity caused by caffeine, and thus, it is possible to improve the circadian rhythm sleep disorders due to a change in the biological cycle caused by caffeine.

In addition, specifically, the form of the food may be coffee, but is not limited thereto. The coffee may be ground coffee or instant coffee, and the form of the coffee is not particularly limited. In addition, specifically, the coffee may include no-caffeine coffee.

The ground coffee may use coffee beans such as Jamaica Blue Mountain, Indonesia Toraja, Colombia Supremo, Guatemala Antigua, Kenya Arabica, Brazil Santos, Ethiopia Mocha Yigacheffe, etc. These coffee beans may be used by drying, roasting, and then grinding green beans, wherein the roasting is a processing process of roasting by heating the green beans and applying heat to the green beans to cause physical and chemical changes in the internal structure of the green beans, thereby determining the unique taste and aroma of coffee.

The instant coffee may further contain sugars, fats and oils, etc. to the coffee beans. At this time, the sugars may use at least one selected from white sugar, glucose, dextrin, fructose, oligosaccharide, propolis, trehalose, sorbitol, xylitol, mannitol, mantitol, rhamnitol, inositol, erythritol, palatinose, and quercitol. In addition, the fats and oils may use at least one selected from powdered milk, skim milk powder, and vegetable powder.

The coffee containing the citrus rind extract of the present invention not only prevents the sleep disorder, which is one of the side effects caused by coffee, but also inhibits neural hyperactivity caused by an overdose of caffeine. In particular, the citrus rind extract is added to or included in coffee so as not to deteriorate the original taste of coffee, and acts competitively with caffeine to minimize an insomnia effect that may be caused by caffeine.

Foods to which the citrus rind extract of the present invention may be added include sausage, meat, bread, chocolates, snacks, candy, confectionery, ramen, pizza, other noodles, gums, dairy products including ice cream, various soups, beverages, teas, drinks, alcoholic beverages and vitamin complexes as well as the coffee. When formulated as beverages, a liquid ingredient to be added in addition to the citrus rind extract of the present invention is not limited thereto, but like general beverages, various flavoring agents, natural carbohydrates, or the like may be contained as an additional ingredient. The natural carbohydrates described above may include monosaccharides (e.g. glucose, fructose, etc.), disaccharides (e.g. maltose, sucrose, etc.), polysaccharides (e.g. general sugars such as dextrin, cyclodextrin, etc.), and sugar alcohols such as xylitol, sorbitol, erythritol, etc.

The type of food may be a food additive, and the food additive refers to a substance to be used by other methods such as adding, mixing, and infiltrating to foods for manufacturing, processing, or preserving foods. The food additive includes natural products and synthetic products, and may be classified according to functions and uses. The form of the food additive may include a powder, granule, tablet, capsule or liquid form, but is not limited thereto.

In addition, the type of food may specifically be a health functional food. The health functional food may contain various nutrients, vitamins, minerals (electrolytes), flavoring agents such as synthetic and natural flavoring agents, coloring agents and enhancers (cheese, chocolate, etc.), pectic acid and salts thereof, organic acid, a protective colloidal thickener, a pH adjusting agent, a stabilizer, a preservative, glycerin, alcohol, a carbonic acid agent used in a carbonated drink, and the like. In addition, the health functional food of the present invention may contain pulp for manufacturing fruit and vegetable beverages. These ingredients may be used alone or in combination, and the ratio of these additives may be selected in the range of 0.001 to 50 parts by weight per total weight of the composition.

The health functional food is a food that emphasizes a biomodulating function of food, and is a food that gives an added value to be applied and expressed for a specific purpose by using physical, biochemical, and bioengineering methods. These ingredients of the health functional food are designed and processed to fully exert the biomodulating functions associated to biodefense, regulation of biorhythm, prevention and recovery of diseases to a living body, and may contain cytological acceptable food additives, sweeteners or functional raw materials.

When the citrus rind extract of the present invention is used as a health functional food (or a health functional beverage additive), the citrus rind extract is added as it is or used with other foods or food ingredients and may be used appropriately depending on a general method. The mixing amount of the citrus rind extract may be appropriately determined according to the purpose of use (prevention, health or improvement, therapeutic treatment).

Another object of the present invention relates to a pharmaceutical composition for preventing or treating sleep disorders caused by caffeine containing a citrus rind extract. More specifically, the sleep disorder may be insomnia or circadian rhythm sleep disorders caused by caffeine.

The "sleep disorder", "insomnia", and "circadian rhythm sleep disorder" are as described above.

For administration, the pharmaceutical composition of the present invention may include a pharmaceutically acceptable carrier, an excipient, or a diluent in addition to the citrus rind extract of the present invention. The carrier, the excipient, and the diluent may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

In addition, the pharmaceutical composition of the present invention is applicable in any formulation, and more specifically, may be an oral formulation. The parenteral formulation may be injections, applications, or spray types such as aerosol.

Yet another aspect of the present invention relates to a method for treating sleep disorders, comprising administering a pharmaceutical composition containing a citrus rind extract to a subject in need thereof in a pharmaceutically effective amount. Specifically, the sleep disorder may be insomnia or circadian rhythm sleep disorder caused by caffeine.

The "sleep disorder", "insomnia", and "circadian rhythm sleep disorder" are as described above.

In the present invention, the "pharmaceutically effective dose" refers to a sufficient amount to treat the diseases at a reasonable benefit/risk ratio applicable to medical treatment. The effective dose level may be determined according to factors including the gender and age of a patient, the type and severity of a disease, the activity of a drug, the sensitivity to a drug, a time of administration, a route of administration, an emission rate, duration of treatment, and simultaneously used drugs, and other factors well-known in the medical field.

The pharmaceutical composition of the present invention may be administered as an individual therapeutic agent or in combination with other therapeutic agents, and sequentially or simultaneously administered with conventional therapeutic agents. In addition, the pharmaceutical composition of the present invention may be administered in single or multiple. It is important to administer an amount capable of obtaining a maximum effect with a minimal amount without side effects in consideration with all the factors, and the amount thereof may be easily determined by those skilled in the art.

The term "subject" of the present invention includes animals or humans having sleep disorders of which symptoms may be improved by administration of the pharmaceutical composition according to the present invention. By administering the therapeutic composition according to the present invention to the subject, it is possible to effectively prevent and treat sleep disorders.

The term "administration" of the present invention means introducing a predetermined substance to humans or animals by any suitable method. The administration route of the therapeutic composition according to the present invention may be administered orally or parenterally through any general route as long as reaching a target tissue. In addition, the therapeutic composition of the present invention may be administered by any device capable of transferring an active ingredient to target cells.

A preferable dose of the pharmaceutical composition of the present invention varies according to the condition and weight of a patient, the degree of a disease, a drug form, and the route and period of administration, but may be properly selected by those skilled in the art.

[Advantageous Effects]

According to the present invention, the composition of the present invention exhibits an effect of improving sleep disorders while sedating excessive neural activity caused by caffeine, and particularly, may also inhibit the long-acting of caffeine beyond an effect of inhibiting the neural activity caused by caffeine in a short period of time. Therefore, the composition of the present invention can be widely used for improving sleep disorders while stabilizing the neural activity induced by caffeine.

[Description of Drawings]

FIG. 1 illustrates a result of confirming ingredients contained in a citrus unshiu peel sample through HPLC.

FIG. 2 illustrates a result of HPLC quantitative analysis of hesperidin contained in the citrus unshiu peel.

FIG. 3 illustrates a result of HPLC quantitative analysis of narirutin contained in the citrus unshiu peel.

FIG. 4 illustrates the contents of hesperidin and narirutin contained in 10 mg of a citrus unshiu peel sample.

FIG. 5 illustrates a hippocampal organotypic slice culture process.

FIG. 6 illustrates a microelectrode array (MEA) setting process of the hippocampal tissue.

FIG. 7 illustrates a microelectrode array (MEA) experimental protocol (A) and a spike detection process (B) using a band pass filter.

FIG. 8 illustrates a result of confirming that the neural activity caused by caffeine is alleviated when a citrus unshiu peel extract is treated (A: neural activity in treatment of caffeine, B: neural activity in treatment of caffeine and citrus unshiu peel extract, C: comparison in neural activity before washing in treatment of caffeine alone and treatment of caffeine and citrus unshiu peel extract, D: comparison in neural activity after washing in treatment of caffeine alone and treatment of caffeine and citrus unshiu peel extract).

FIG. 9 illustrates a result of confirming that the neural activity caused by caffeine is alleviated when hesperidin, narirutin, or a mixture thereof contained in the citrus unshiu peel extract is treated (A: neural activity in treatment of caffeine, B: neural activity in treatment of caffeine and hesperidin, C: neural activity in treatment of caffeine and narirutin, D: neural activity in treatment of caffeine, hesperidin, and narirutin).

FIG. 10 illustrates comparison of a result before and after washing of confirming that the neural activity caused by caffeine is alleviated when hesperidin, narirutin, or a mixture thereof contained in the citrus unshiu peel extract is treated (A: before caffeine washing, B: after caffeine washing).

FIG. 11 illustrates an experimental process of oral administration and recording to an SD rat for measuring sleep duration (A: oral administration process to SD rat, B: recording process for measuring sleep duration).

FIG. 12 illustrates a result of confirming that the sleep duration is recovered when the citrus unshiu peel extract is treated.

**[Best Mode of the Invention]**

Hereinafter, the present invention will be described in detail by Examples. However, the following Examples are just illustrative of the present invention, and the contents of the present invention are not limited to the following Examples.

**Preparation Example. Preparation of citrus unshiu peel extract**

100 g of a citrus unshiu peel (Gio Herb Co., Yangju, Korea) was added with 10 times (w/v) of distilled water and extracted by hot water at 121°C for 40 minutes. The extract was filtered using Whatman No. 1 filter paper (Whatman International Ltd., Maidstone, UK), and the filtered extract was concentrated for 5 hours at 40°C using a concentrator (Eyela Co., Tokyo, Japan). The concentrated extract was freeze-dried for 7 days at - 70°C and 5 mTorr with a freeze dryer (Ilsin Bio Co., Yangju., Korea), and then grinded with a grinder (Ilsin Bio Co., Yangju., Korea) and used for an experiment. When the citrus unshiu peel was extracted with hot water through the above process, the yield was 9.8%.

[Table 1]

| | Raw material (g) | Extract (g) | Yield (%; extract/raw material) |
|---|---|---|---|
| Citrus unshiu peel | 100 | 9.8 | 9.8 |

**Experimental Example 1. Confirmation of ingredients contained in citrus unshiu peel extract**

Compounds contained in the citrus unshiu peel extract of the present invention were confirmed using HPLC. Specifically, the citrus unshiu peel extract was measured using reverse phase HPLC (Vltimate 3000; Thermo Fisher Scientific, Waltham, USA) consisting of an autosampler, a binary pump, and a PDA detector. The reverse phase HPLC used C18 (Discovery C18, 5 $\mu$m, 4.6 $\times$ 250 mm; Sigma, MO, USA). An injection amount thereof was 10 $\mu$L and injected at a rate of 0.8 mL/min.

In the HPLC, solvents (mobile phase) A and B used 100% tertiary distilled water and 100% acetonitrile, respectively, and the composition of gradient elution was performed at 90% A/10% B in 0 to 5 minutes, 75% A/25% B in 5 to 14 minutes, 40% A/60% B in 14 to 25.5 minutes, 20% A/80% B in 25.5 to 30.5 minutes, and 90% A/10% B in 30.5 to 35 minutes. All compounds were detected at a wavelength of 280 nm, and the analysis temperature was maintained at 40°C. The data of a sample test solution collected through HPLC/PDA was confirmed by comparing a retention time and a UV spectrum waveform of a standard form.

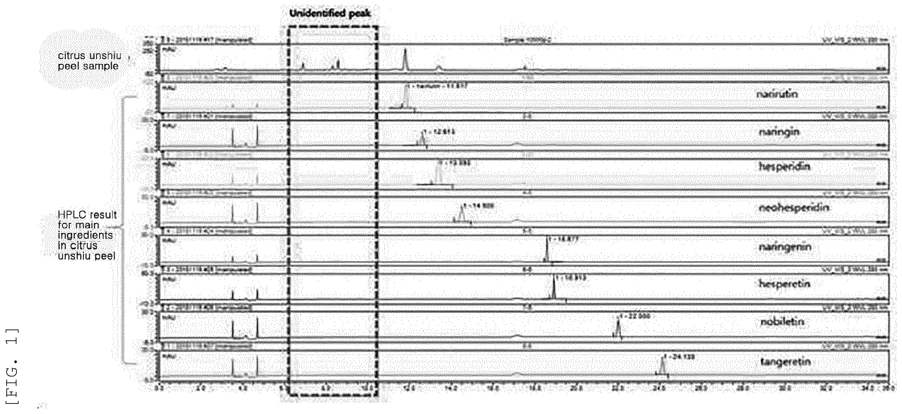

As a result, as illustrated in FIG. 1, it was confirmed that narirutin and hesperidin were contained in the citrus unshiu peel sample used in the present invention.

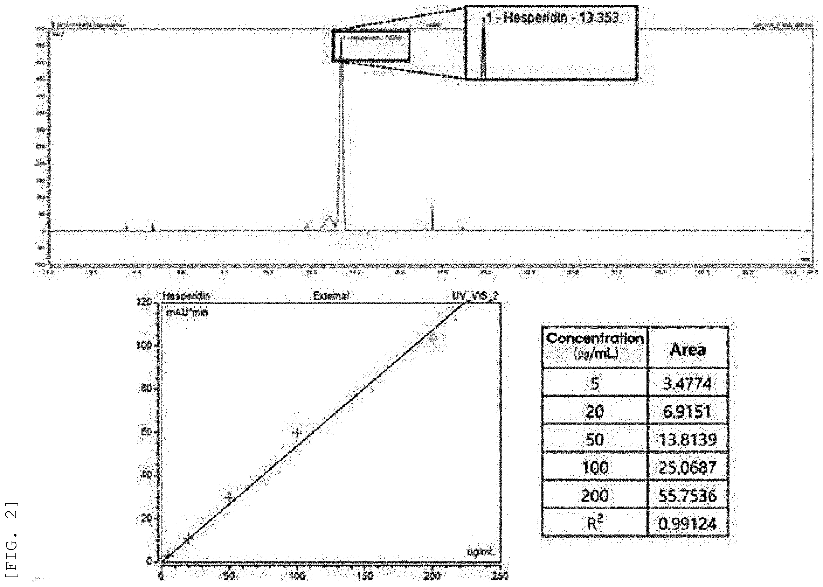

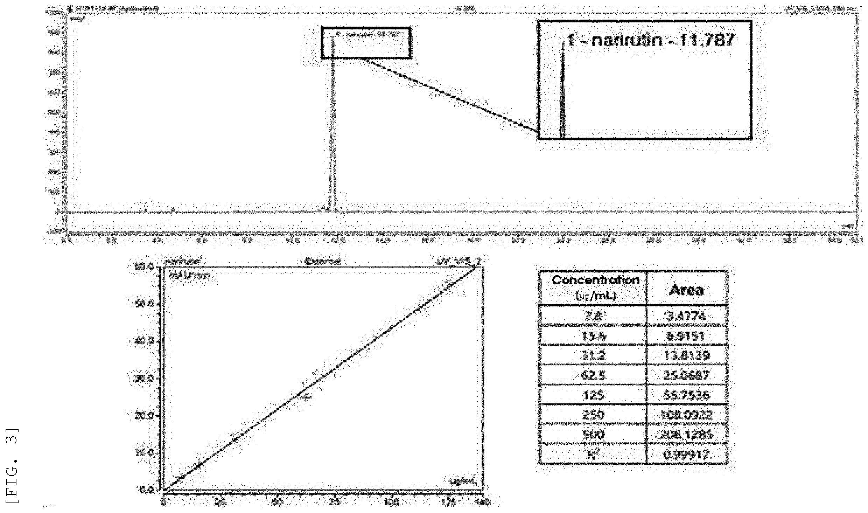

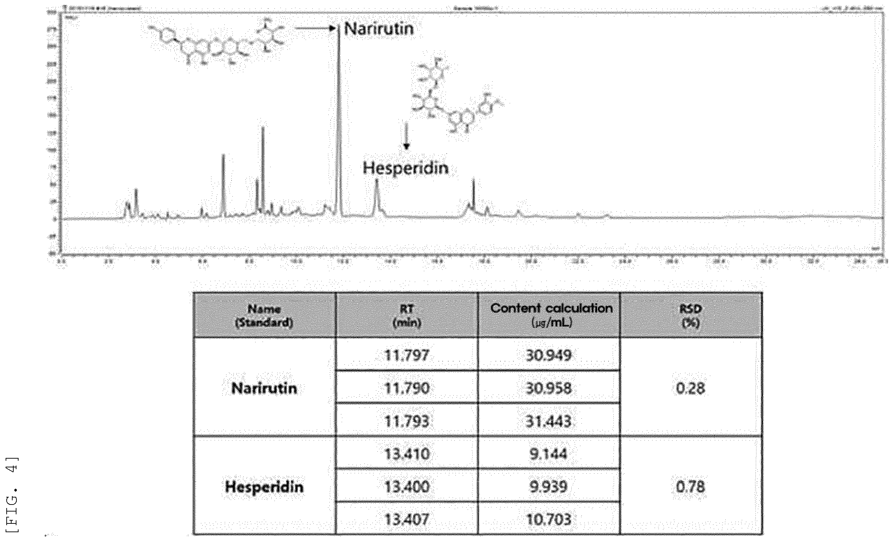

In addition, the contents of narirutin and hesperidin in the citrus unshiu peel extract were calculated (FIGS. 2 and 3), and as a result, it was confirmed that the contents of narirutin and hesperidin contained in 400 $\mu$g/ml of the citrus unshiu peel extract were 136 $\mu$g/ml and 40 $\mu$g/ml, respectively (FIG. 4).

**Experimental Example 2. Measurement of neural activity**

1-1. Hippocampal organotypic slice culture (HOSC)

A method of organotypic culturing of slices of the brain hippocampus using a thin film used an already commonly used method. Specifically, the brain hippocampus was extracted from a Sprague Dawley white young rat (Orientbio Co., Seungnam, Korea) of 5- to 7-day old, and sliced to a thickness of 350 $\mu$m using a tissue slicer (Mickle Laboratory Engineering Co., UK). After 5 to 6 slices were disposed into a thin film insert (Millicell-CM; Millipore, MA) with 0.4-$\mu$m holes, put in a 6-well plate (SPL Life Sciences Co., Korea), and then cultured in an incubator kept with 5% $CO_2$ at 36°C. (Dong-A Co., Seoul, Korea). A culture solution (MEM; Welgene, Gyeongsan, South Korea), 20 mM HEPES (Sigma, St. Louis, Mo, USA), a 25% Hank's balanced salt solution (HBSS; Welgene, Gyeongsan, South Korea), 6 g/L D-glucose (Sigma, St. Louis, Mo, USA), 1 mM L-glutamine (Sigma, St. Louis, Mo, USA), 25% horse serum (Welgene, Gyeongsan, South Korea), and 1% penicillin-streptomycin (Gibco BRL, USA), pH=7.1) were changed once every two days, and the culture period was 12 to 14 days, and then used in a subsequent experiment (FIG. 5).

1-2. Migration of brain hippocampal tissue cultured with MEA probes

After the cultured brain hippocampal slices were carefully isolated from the cultured thin film insert using a pin, the brain hippocampal slices were placed on a 8 x 8 microelectrode array (MEA; Multi Channel Systems, Germany) with a diameter of 10 $\mu$m and an interval of 100 $\mu$m coated with 0.01% polyethyleneimine (Sigma, St. Louis, Mo, USA). The slices were injected with a sterilized artificial spinal cord solution (aCSF; 114 mM NaCl, 26 mM $NaHCO_3$, 10 mM glucose, 3 mM KCl, 2 mM CaCl2, 1 mM MgCl2, 20 mM HEPES, pH=7.4, Sigma, Louis, Mo, USA) at 5% $CO_2$ and 95% $O_2$ mixed gas (Dong-A Co., Seoul, Korea) for 15 minutes and stabilized while kept at 33°C. Thereafter, the MEA containing the brain hippocampus was mounted on an amplification device MEA1060 (Multi Channel Systems Inc., Reutlingen, Germany), and the artificial spinal cord solution in the MEA was grounded with Ag/AgCl. During the subsequent experiment, the artificial spinal cord solution into which the 5% $CO_2$ and 95% $O_2$ mixed gas was added was perfused at a rate of 3 mL/min, and the MEA and the artificial spinal cord solution were maintained at 33°C (FIG. 6).

1-3. Measurement of neural activity

The stabilized hippocampal tissue was recorded for 3 minutes, and then after an artificial cerebrospinal fluid containing 1 mM of caffeine (Sigma, St. Louis, Mo, USA) and an artificial cerebrospinal fluid containing 1 mM of caffeine and an analysis sample (citrus unshiu peel extract of 400 $\mu$g/ml, hesperidin of 40 $\mu$g/ml, narirutin 136 of $\mu$g/ml) were perfused under the same conditions for 20 minutes, respectively, the hippocampal tissue was recorded for 3 minutes. Thereafter, the hippocampal tissue was washed with only the artificial cerebrospinal fluid for 20 minutes and then recorded for 3 minutes to obtain data for total 9 minutes.

The MEA with the hippocampal tissue read data at a sampling rate of 25 kHz for each channel through a total of 60 channels, and was recorded through a computer using MC_Rack and MC_Data Tool (Multi Channel Systems, GmbH) software. A 4-stage band pass filter was used to extract meaningful spikes from all recorded signals, and Equation is as follows.

[Equation 1]

$$Threshold = k \times median\left(\frac{abs(X)}{0.6745}\right)$$

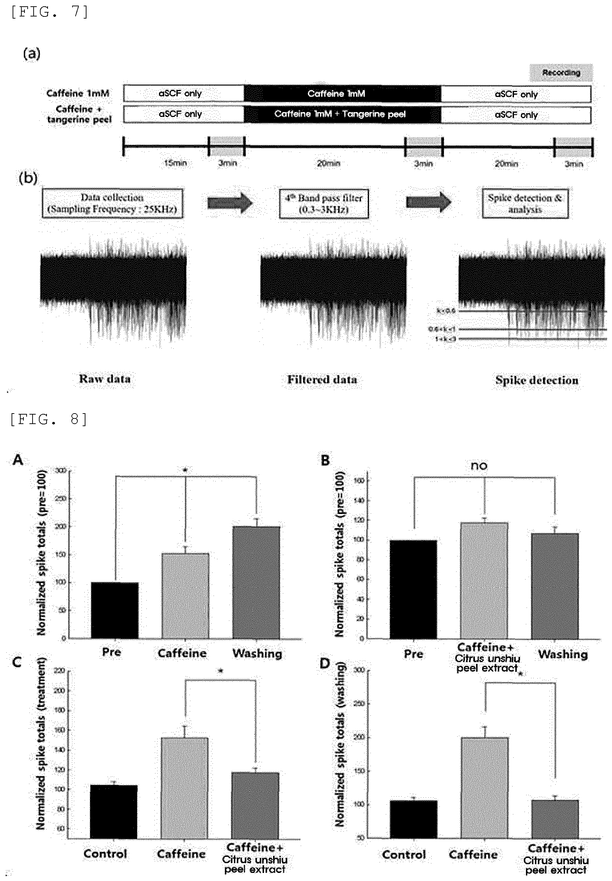

Wherein, X represents a root mean square (RMS) for noise, and k is a factor that determines each threshold. Here, the threshold consisted of a total of three factors (0.6, 1 and 3). When the k value was 0.6 or higher, a meaningful spike was used, and the threshold level at this time was - 21.63 $\mu$V. This process was performed using a MATLAB (Mathworks Inc., Natick, Massachusetts, USA) program for all 60 channels (FIG. 7).

As a result, as illustrated in FIG. 8, it was confirmed that the neural activity caused by caffeine was alleviated when the citrus unshiu peel extract was treated.

Specifically, when caffeine was treated, the neural activity was increased, and even after washing after the caffeine was treated, the action of caffeine continued to increase the neural activity by 200% or more (FIG. 8A). On the other hand, it was confirmed that when the caffeine and the citrus unshiu peel extract were treated together, the degree of neural activity was significantly reduced compared to the case of treatment of caffeine alone, and unlike treatment of caffeine alone, the neural activity was not increased continuously even after washing, and thus, the neural activity was maintained without a significant increase (FIG. 8B). With respect to the case of treating caffeine alone and the case of treating caffeine and the citrus unshiu peel extract in combination, even when comparing before and after washing, in treatment with the citrus unshiu peel extract, it was confirmed that the significant neural activity reduction effect was shown (FIGS. 8C and 8D).

In particular, even after washing after treatment with caffeine, the activity caused by caffeine in the nerves was increased, and this indicates that the active action of caffeine on the nerves does not appear briefly in a short time, but rather continues. The citrus unshiu peel extract of the present invention exhibited the effect of reducing the neural activity by inhibiting the effect of caffeine which was continuously increased as described above.

In addition, as a result of treating hesperidin, narirutin, or a mixture thereof, which were ingredients contained in the citrus unshiu peel extract, together with caffeine, it was confirmed that both hesperidin and narirutin may inhibit an increase in neural activity caused by caffeine. Furthermore, it was confirmed that the neural activity was more effectively inhibited when hesperidin and narirutin were treated in combination than when each of hesperidin and narirutin was treated (FIG. 9).

Specifically, when the caffeine was treated, the neural activity was increased, and even after washing after the caffeine was treated, the action of caffeine continued to increase the neural activity by 200% or more (FIG. 9A). On the other hand, it was confirmed that when the caffeine and 40 $\mu$g/ml of hesperidin (amount contained in 400 $\mu$g/ml of the citrus unshiu peel) were treated in combination, the degree of neural activity was significantly reduced compared to the case of treatment of caffeine alone, and unlike treatment of caffeine alone, the neural activity was not increased continuously even after washing, and thus, the neural activity was maintained without a significant increase (FIG. 9B). It was confirmed that even when the caffeine and 136 $\mu$g/ml of narirutin (amount contained in 400 $\mu$g/ml of citrus unshiu peel) were treated in combination, similarly, the degree of neural activity was significantly reduced compared to the case of treatment of caffeine alone, and unlike treatment of caffeine alone, the neural activity was not increased continuously even after washing, and thus, the neural activity was maintained without a significant increase (FIG. 9C). Further, it was confirmed that the neural activity reduction effect was further increased when 40 $\mu$g/ml of hesperidin and 136 $\mu$g/ml of narirutin were treated together with caffeine (FIG. 9D).

Through the results, it was confirmed that the hesperidin and narirutin ingredients may alleviate the neural activity, and the citrus unshiu peel extract containing both the two ingredients may effectively alleviate the neural activity caused by caffeine.

Furthermore, as illustrated in FIG. 10, when caffeine is treated, the neural activity is continuously shown even after washing the caffeine, and this indicates that the neural activity action caused by caffeine may continue for a long time rather than a short time and there is a possibility of side effects as the neural activity continues for a long time.

On the other hand, unlike the case of treatment of caffeine alone, it was confirmed that when hesperidin, narirutin, and a mixture of hesperidin and narirutin were treated in combination, a neural activity inhibitory effect persisted even after washing. This indicates that the neural activity induced by caffeine may be inhibited from continuing even after caffeine is removed (FIG. 10).

**Experimental Example 3. Confirmation of effect of improving sleep disorders**

3-1. Experimental Animals

A 6-week-old Sprague Dawley rat was selected as an experimental animal, and was purchased from Orient Bio Co., Ltd. (Seongnam, Korea). In order to keep an experimental environment constant, an experimental animal per cage was placed and bred one by one. The experimental animals went through a period of adjustment for one day in an environment where 25°C, humidity 50%, and lighting (12 hours light/dark) were automatically maintained and drinking water and diet were supplied freely. All processes followed the policy of Kyunghee University's Experimental Animal Ethics Committee and the regulations associated to Animal Experiment (IRB approval number: KHUASP(SE)-17-039).

2-2. Measurement of sleep duration

The experiment was performed by dividing 15 SD rats into 3 groups (n = 5). Water was orally administered to Group 1, caffeine (20 mg/Kg) was orally administered to Group 2, and a citrus unshiu peel extract (100 mg/Kg) and caffeine (20 mg/Kg) were orally administered to Group 3 together.

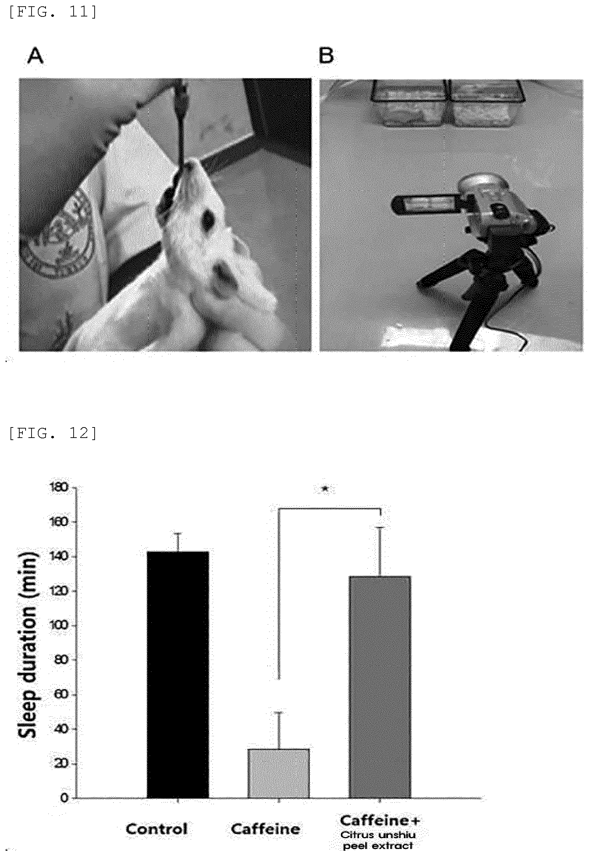

Focusing on the fact that rodents were nocturnal in nature, the sleep duration was measured during daytime when SD rats took sleep originally. After the oral administration, the cage was recorded for 5 hours using a camcorder (Sony Co., Tokyo, Japan) from 10 a.m. to measure the sleep duration, and the measurement time was referred to in previous studies. A total of 3 subjects were recorded one by one for each group per day, and only one subject for each cage was placed so that the subjects were not affected by each other. In addition, the illuminance of a space to be measured was kept constant, and humidity 50%, temperature 25°C, and sound insulation were maintained. The sleep pattern and duration were measured using a MultiChannel Stopwatch (Techsys Co. Seoul, Korea) by determining as a sleep condition when the rat closed eyes and continued for 5 minutes or more in a prone position on the side and summing hours from the start of sleep to the end of sleep (FIG. 11).

As a result, as illustrated in FIG. 12, when the caffeine was treated, the sleep duration was reduced to about 20 to 30% compared to a normal control, but when the caffeine and the citrus unshiu peel extract were treated together, the sleep duration was restored by about 4 times or higher.

The results described above confirmed that the citrus unshiu peel extract of the present invention had an effect of improving sleep disorders while sedating the excessive neural activity caused by caffeine, and particularly, it was shown that the citrus unshiu peel extract may inhibit the continuous action of caffeine beyond the effect of inhibiting the neural activity caused by caffeine in a short time. Accordingly, the citrus unshiu peel extract of the present invention may be widely used for improving sleep disorders while stabilizing neural activity induced by caffeine.

The results of the experiment were expressed as mean $\pm$ standard error (S.E.M). After converting the calculated spike values into ratios thereof in each group, an ANOVA Tukey post-test was performed to examine differences between groups. At $p < 0.05$, $p < 0.01$, and $p < 0.001$, there were statistically significant differences between groups.

**Experimental Example 4. Confirmation of anti-insomnia activity of citrus rind extract**

In order to confirm whether the citrus rind extract has an anti-insomnia effect, with respect to mandarin, tangerine, sweetie, citron, citrus sudachi, cheonhyehyang, calamansi, pomelo, hanrabong, orange, lemon, grapefruit, lime, and yuja rind extracts, the experiments of Experimental Examples 2 and 3 were repeated.

As a result of the experiments, each of the rind extracts had some differences from the citrus unshiu peel extract of Example 1, but a similar or equivalent level of sleep disorder improvement effect was implemented. Through this, it was confirmed that the citrus rind extracts had similar properties and exhibited an anti-insomnia effect in common.

The aforementioned description of the present invention is to be exemplified, and it will be understood to those skilled in the art that the technical spirit or required features of the present invention can be easily modified in other detailed forms without changing. Therefore, it should be appreciated that the aforementioned embodiments described above are illustrative in all aspects and are not restricted. For example, respective components described as single types can be distributed and implemented, and similarly, components described to be distributed can also be implemented in a coupled form.

The scope of the present invention is represented by claims to be described below, and it is to be interpreted that the meaning and the scope of the appended claims and all changed or modified forms derived from equivalents thereof come within the scope of the present invention.

**Claims**

1.  A food composition for preventing or improving sleep disorders caused by caffeine containing a citrus rind extract.

2.  The food composition of claim 1, wherein the citrus is at least one selected from the group consisting of clementine, kumquat, mandarin, tangerine, sweetie, citron, citrus sudachi, cheonhyehyang, calamansi, pomelo, hanrabong, orange, lemon, grapefruit, lime, and yuja.

3.  The food composition of claim 1, wherein the citrus rind extract contains at least one of hesperidin and narirutin.

4.  The food composition of claim 1, wherein the extract is extracted with water, alcohol, ethyl acetate, acetone, nucleic acid, dichloromethane, or a mixed solvent thereof.

5.  The food composition of claim 1, wherein the food composition is coffee.

6.  The food composition of claim 1, wherein the food composition is food additives.

7.  The food composition of claim 1, wherein the sleep disorders are insomnia or circadian rhythm sleep disorders caused by caffeine.

8.  A pharmaceutical composition for preventing and treating sleep disorders caused by caffeine containing a citrus rind extract.

9. A method for preventing, improving, or treating sleep disorders caused by caffeine comprising administering a citrus rind extract to a subject.

[FIG. 1]

[FIG. 2]

[FIG. 3]

| Concentration (µg/mL) | Area |
|---|---|
| 7.8 | 3.4774 |
| 15.6 | 6.9151 |
| 31.2 | 13.8139 |
| 62.5 | 25.0687 |
| 125 | 55.7536 |
| 250 | 108.0922 |
| 500 | 206.1285 |
| R² | 0.99917 |

1 - narirutin - 11.787

[FIG. 4]

| Name (Standard) | RT (min) | Content calculation (µg/mL) | RSD (%) |
|---|---|---|---|
| Narirutin | 11.797 | 30.949 | 0.28 |
| | 11.790 | 30.958 | |
| | 11.793 | 31.443 | |
| Hesperidin | 13.410 | 9.144 | 0.78 |
| | 13.400 | 9.939 | |
| | 13.407 | 10.703 | |

Narirutin

Hesperidin

[FIG. 5]

[FIG. 6]

[FIG. 7]

(a)

(b)

Raw data　　　　　Filtered data　　　　　Spike detection

[FIG. 8]

[FIG. 9]

[FIG. 10]

[FIG. 11]

[FIG. 12]

| INTERNATIONAL SEARCH REPORT | International application No. |
|---|---|
| | PCT/KR2019/095002 |

| A. CLASSIFICATION OF SUBJECT MATTER |
|---|
| *A23L 33/105(2016.01)i, A23L 19/00(2016.01)i, A23F 5/24(2006.01)i* |
| According to International Patent Classification (IPC) or to both national classification and IPC |

| B. FIELDS SEARCHED |
|---|
| Minimum documentation searched (classification system followed by classification symbols) |
| A23L 33/105; A23F 5/00; A23F 5/14; A23L 2/02; A23L 2/38; A61K 31/015; A61K 31/7048; A61K 35/78; A61K 36/752; A61P 25/30; A23L 19/00; A23F 5/24 |
| Documentation searched other than minimum documentation to the extent that such documents are included in the fields searched<br>Korean utility models and applications for utility models: IPC as above<br>Japanese utility models and applications for utility models: IPC as above |
| Electronic data base consulted during the international search (name of data base and, where practicable, search terms used)<br>eKOMPASS (KIPO internal) & Keywords: citrus, caffeine, sleep disorder, Hesperidin, narirutin, coffee |

| C. DOCUMENTS CONSIDERED TO BE RELEVANT |
|---|

| Category* | Citation of document, with indication, where appropriate, of the relevant passages | Relevant to claim No. |
|---|---|---|
| Y | KR 10-2011-0120153 A (KOREA UNIVERSITY RESEARCH AND BUSINESS FOUNDATION) 03 November 2011<br>See paragraphs [0004], [0013], [0021], [0022]; and claims 1-3. | 1-8 |
| Y | HUANG, Z.-L. et al. Adenosine A2A, but not A1, receptors mediate the arousal effect of caffeine. Nature Neuroscience. 2005, vol. 8, no. 7, pages 858-859<br>See pages 858, 859; and figures 1, 2. | 1-8 |
| Y | KR 10-0857003 B1 (OH, Kyong Duk) 04 September 2008<br>See paragraphs [0003], [0008]. | 3 |
| Y | KR 10-2013-0035530 A (KOREA FOOD RESEARCH INSTITUTE) 09 April 2013<br>See claims 8-15. | 3 |
| A | WO 02-066041 A1 (HERBAL DETOX THERAPY APS.) 29 August 2002<br>See claims 12-14, 41, 76. | 1-8 |
| A | KR 10-1014568 B1 (GOH, Bu Eon et al.) 16 February 2011<br>See the entire document. | 1-8 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 26 AUGUST 2019 (26.08.2019) | **27 AUGUST 2019 (27.08.2019)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

<table>
<tr><td>INTERNATIONAL SEARCH REPORT</td><td>International application No.<br><br>PCT/KR2019/095002</td></tr>
</table>

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 9
because they relate to subject matter not required to be searched by this Authority, namely:

Claim 9 pertains to a method for treatment of the human body by surgery or therapy, as well as a diagnostic method, and thus pertains to a subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT
Information on patent family members

International application No.

**PCT/KR2019/095002**

| Patent document cited in search report | Publication date | Patent family member | Publication date |
|---|---|---|---|
| KR 10-2011-0120153 A | 03/11/2011 | None | |
| KR 10-0857003 B1 | 04/09/2008 | None | |
| KR 10-2013-0035530 A | 09/04/2013 | KR 10-1301971 B1 | 30/08/2013 |
| | | US 2014-0364382 A1 | 11/12/2014 |
| | | US 9724361 B2 | 08/08/2017 |
| | | WO 2013-047958 A1 | 04/04/2013 |
| WO 02-066041 A1 | 29/08/2002 | None | |
| KR 10-1014568 B1 | 16/02/2011 | KR 10-2010-0052803 A | 20/05/2010 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101265253